# EUROPEAN PATENT APPLICATION

(11) **EP 4 850 809 A1**
(43) Date of publication of application: **09.12.2026**
(21) Application number: 25748338.8
(22) Date of filing: 16.01.2025
(51) Int. Cl.: B01J 20/26, A61F 13/15, A61F 13/53, C08F 2/10, C08J 3/12

(54) **WATER ABSORBENT RESIN PARTICLES, ABSORBER, AND ABSORBENT ARTICLE**

(30) Priority: 29.01.2024 JP 2024011357
(71) Applicant: Sumitomo Seika Chemicals Co., Ltd., Kako-gun, Hyogo 675-0145 (JP)
(72) Inventor: HIRANO, Saho, Himeji-shi, Hyogo 672-8076 (JP)
(74) Representative: Mewburn Ellis LLP
(86) International application number: PCT/JP2025/001232
(87) International publication number: WO 2025/164342

(57) **Abstract**

The present invention provides water-absorbent resin particles in which, when the water-absorbent resin particles are applied to an absorbent material, re-wet of a liquid after absorption of the liquid by the absorbent material that is in a curved state and under pressure is prevented. Water-absorbent resin particles having a value of physiological saline absorption capacity under a load of 0.69 kPa after 15 minutes of 30 mL/g or more and 60 mL/g or less.

## Description

### Technical Field

The present invention relates to water-absorbent resin particles, an absorbent material, and an absorbent article; and more particularly to water-absorbent resin particles constituting an absorbent material suitably used for hygienic materials, such as disposable diapers, sanitary napkins, and incontinence pads, as well as an absorbent material and an absorbent article obtained using the water-absorbent resin particles.

### Background Art

In recent years, water-absorbent resin particles have been widely used in the field of hygienic materials, such as disposable diapers, sanitary napkins, and incontinence pads.

As such water-absorbent resin particles, crosslinked products of polymers of a water-soluble ethylenically unsaturated monomer, more specifically, crosslinked products of polymers of partially neutralized polyacrylic acid, have been proposed as preferable water-absorbent resin particles, because of many advantages: for example, they have good water absorption capacity; acrylic acid used as a raw material is readily industrially available, and thus, they can be produced at low cost with uniform quality; additionally, they are resistant to decomposition or degradation (see, for example, Patent Literature 1).

An absorbent article, such as a disposable diaper, a sanitary napkin, or an incontinence pad, is composed of an absorbent material that is positioned mainly in a central portion to absorb and retain a body liquid, such as urine or menses excreted from the body, a liquid-permeable front sheet (top sheet) positioned on the side of the absorbent article that is brought into contact with the body, and a liquid-impermeable rear sheet (back sheet) positioned opposite to the side that is brought into contact with the body. The absorbent material is typically composed of hydrophilic fibers, such as pulp, and water-absorbent resin particles.

### Citation List

### Patent Literature

Patent Literature 1: JP-A-H3-227301

### Summary of Invention

### Technical Problem

In such an absorbent article, after the absorbent material absorbs a liquid while the absorbent material is in a curved state or under pressure, the liquid may return from the absorbent material to the wearer side of the diaper, that is, so-called re-wet may occur.

The present inventor has intensively studied a technique for preventing the re-wet after absorption of a liquid by the absorbent material that is in a curved state or a technique for preventing the re-wet after absorption of a liquid by the absorbent material that is under pressure. Specifically, in light of prior art, the present inventor has studied techniques for preventing the re-wet of a liquid by adjusting various characteristics of water-absorbent resin particles, such as physiological saline absorption capacity under a load, non-pressurization DW value, water absorption rate with respect to physiological saline, and physiological saline retention capacity of the water-absorbent resin particles. However, although the present inventor applied these techniques to the absorbent material, it was difficult to sufficiently prevent the re-wet when the absorbent material absorbed a liquid while the absorbent material was in a curved state and under pressure.

Under such circumstances, a main object of the present invention is to provide water-absorbent resin particles in which, when the water-absorbent resin particles are applied to an absorbent material, re-wet of a liquid after absorption of the liquid by the absorbent material that is in a curved state and under pressure is prevented. Another object of the present invention is to provide an absorbent material and an absorbent article obtained using the water-absorbent resin particles.

### Solution to Problem

The present inventor conducted extensive research to solve the above problem. As a result, the present inventor found that by setting a value of physiological saline absorption capacity under a load of 0.69 kPa after 15 minutes of water-absorbent resin particles in a specific range of 30 mL/g or more and 60 mL/g or less, when the water-absorbent resin particles are applied to an absorbent material, re-wet of a liquid after absorption of the liquid by the absorbent material that is in a curved state and under pressure is prevented. The present invention has been completed as a result of further research based on this finding.

In summary, the present invention provides aspects of the invention comprising the following features:
Item 1. Water-absorbent resin particles having a value of physiological saline absorption capacity under a load of 0.69 kPa after 15 minutes of 30 mL/g or more and 60 mL/g or less.
Item 2. The water-absorbent resin particles according to Item 1, wherein the water-absorbent resin particles have a value of non-pressurization DW after 3 minutes of 20 mL/g or more and 50 mL/g or less.
Item 3. The water-absorbent resin particles according to Item 2, wherein the water-absorbent resin particles have a water absorption rate with respect to physiological saline of 60 seconds or less.
Item 4. The water-absorbent resin particles according to Item 2, wherein the water-absorbent resin particles have a physiological saline retention capacity of 30 g/g or more.
Item 5. An absorbent material comprising the water-absorbent resin particles according to any one of Items 1 to 4.
Item 6. An absorbent article comprising the absorbent material according to Item 5.

### Advantageous Effects of Invention

According to the present invention, it is possible to provide water-absorbent resin particles in which, when the water-absorbent resin particles are applied to an absorbent material, re-wet of a liquid after absorption of the liquid by the absorbent material that is in a curved state and under pressure is prevented. According to the present invention, it is also possible to provide an absorbent material and an absorbent article obtained using the water-absorbent resin particles.

### Brief Description of Drawings

Fig. 1 is a schematic diagram of a measurement apparatus for physiological saline absorption capacity under a load of 0.69 kPa.
Fig. 2 is a schematic diagram of an apparatus for measuring values of non-pressurization DW after 3 and 10 minutes of the water-absorbent resin particles.

### Description of Embodiments

As used herein, the term "comprising" includes "consisting essentially of" and "consisting of". As used herein, the term "(meth)acrylic" refers to "acrylic or methacrylic", the term "(meth)acrylate" refers to "acrylate or methacrylate", and the term "(poly)" means both cases with and without the prefix "poly". As used herein, the term "water-soluble" refers to having a water solubility of 5% by mass or more at 25°C.

As used herein, values combined with "to" refer to the numerical range including the values before and after "to" as the lower and upper limits. When a plurality of lower limits and a plurality of upper limits are mentioned separately, any lower limit and any upper limit may be selected and combined with "to".

### 1. Water-Absorbent Resin Particles

Water-absorbent resin particles of the present invention have a value of physiological saline absorption capacity under a load of 0.69 kPa after 15 minutes of 30 mL/g or more and 60 mL/g or less. Because of this feature of the water-absorbent resin particles of the present invention, when the water-absorbent resin particles are applied to an absorbent material, re-wet of a liquid after absorption of the liquid by the absorbent material that is in a curved state and under pressure is prevented. The water-absorbent resin particles of the present invention will be described below in detail.

As described above, in light of prior art, the present inventor studied techniques for preventing the re-wet of a liquid by adjusting various characteristics of water-absorbent resin particles, such as physiological saline absorption capacity under a load, non-pressurization DW value, water absorption rate with respect to physiological saline, and physiological saline retention capacity of the water-absorbent resin particles. However, although the present inventor applied these techniques to the absorbent material, it was difficult to sufficiently prevent the re-wet when the absorbent material absorbed a liquid while the absorbent material was in a curved state and under pressure. To solve this, the present inventor focused on the value of physiological saline absorption capacity under a load of 0.69 kPa after 15 minutes of the water-absorbent resin particles, and found that by setting the value in the specific range of 30 mL/g or more and 60 mL/g or less, when the water-absorbent resin particles are applied to an absorbent material, re-wet of a liquid after absorption of the liquid by the absorbent material that is in a curved state and under pressure is prevented. This mechanism can be explained, for example, as follows. In the absorbent article with the absorbent material being in a curved state and under pressure, the actual load applied to the water-absorbent resin particles is lighter than the load applied to the absorbent article, due to the influence of the liquid-permeable front sheet (top sheet), hydrophilic fibers, and the like. Thus, it is assumed that the re-wet is prevented by setting the value of physiological saline absorption capacity under a relatively low load condition, that is, under a load of 0.69 kPa, after 15 minutes in the predetermined range, in the water-absorbent resin particles.

In view of more suitably achieving the effect of the present invention, the value of physiological saline absorption capacity under a load of 0.69 kPa after 15 minutes of the water-absorbent resin particles is preferably 30 mL/g or more, more preferably 32 mL/g or more, still more preferably 34 mL/g or more, and particularly preferably 35 mL/g or more; and is preferably 60 mL/g or less, more preferably 58 mL/g or less, and still more preferably 55 mL/g or less. Preferred ranges include from 30 to 60 mL/g, from 30 to 58 mL/g, from 30 to 55 mL/g, from 32 to 60 mL/g, from 32 to 58 mL/g, from 32 to 55 mL/, from 34 to 60 mL/g, from 34 to 58 mL/g, from 34 to 55 mL/g, from 35 to 60 mL/g, from 35 to 58 mL/g, and from 35 to 55 mL/g.

The value of physiological saline absorption capacity under a load of 0.69 kPa after 15 minutes of the water-absorbent resin particles is measured by the method described in the Examples section.

Examples of methods of adjusting the value of physiological saline absorption capacity under a load of 0.69 kPa after 15 minutes of the water-absorbent resin particles include increasing the amount of surface-crosslinking agent used in the method for producing the water-absorbent resin particles described below; and increasing the ratio of a water-soluble ethylenically unsaturated monomer in a second-stage aqueous monomer solution to a water-soluble ethylenically unsaturated monomer in a first-stage aqueous monomer solution. These methods can strengthen the surface-crosslinked layer of the water-absorbent resin particles, so that the liquid can be suitably absorbed and retained under a low load condition of 0.69 kPa and in a short time that is the value after 15 minutes.

In view of increasing the water absorption rate (reducing the absorption time) of the absorbent material while more suitably achieving the effect of the present invention, a value of non-pressurization DW after 3 minutes of the water-absorbent resin particles is preferably 20 mL/g or more, more preferably 30 mL/g or more, still more preferably 33 mL/g or more, particularly preferably 36 mL/g or more, and more particularly preferably 39 mL/g or more; and is preferably 50 mL/g or less, more preferably 48 mL/g or less, and still more preferably 46 mL/g or less. Preferred ranges include from 20 to 50 mL/g, from 20 to 48 mL/g, from 20 to 46 mL/g, from 30 to 50 mL/g, from 30 to 48 mL/g, from 30 to 46 mL/g, from 33 to 50 mL/g, from 33 to 48 mL/g, from 33 to 46 mL/g, from 36 to 50 mL/g, from 36 to 48 mL/g, from 36 to 46 mL/g, from 39 to 50 mL/g, from 39 to 48 mL/g, and from 39 to 46 mL/g.

In view of more suitably achieving the effect of the present invention, a value of non-pressurization DW after 10 minutes of the water-absorbent resin particles is preferably 30 mL/g or more, more preferably 45 mL/g or more, still more preferably 50 mL/g or more, particularly preferably 54 mL/g or more, and more particularly preferably 56 mL/g or more; and is preferably 80 mL/g or less, more preferably 75 mL/g or less, and still more preferably 72 mL/g or less. Preferred ranges include from 30 to 80 mL/g, from 30 to 75 mL/g, from 30 to 72 mL/g, from 45 to 80 mL/g, from 45 to 75 mL/g, from 45 to 72 mL/g, from 50 to 80 mL/g, from 50 to 75 mL/g, from 50 to 72 mL/g, from 54 to 80 mL/g, from 54 to 75 mL/g, from 54 to 72 mL/g, from 56 to 80 mL/g, from 56 to 75 mL/g, and from 56 to 72 mL/g.

The values of non-pressurization DW after 3 and 10 minutes of the water-absorbent resin particles are each measured by the method described in the Examples section.

In view of increasing the water absorption rate (reducing the absorption time) of the absorbent material while more suitably achieving the effect of the present invention, a water absorption rate with respect to physiological saline of the water-absorbent resin particles is preferably 60 seconds or less, more preferably 55 seconds or less, and still more preferably 50 seconds or less; and is preferably 20 seconds or more, more preferably 25 seconds or more, and still more preferably 30 seconds or more. Preferred ranges include from 20 to 60 seconds, from 20 to 55 seconds, from 20 to 50 seconds, from 25 to 60 seconds, from 25 to 55 seconds, from 25 to 50 seconds, from 30 to 60 seconds, from 30 to 55 seconds, and from 30 to 50 seconds.

The water absorption rate with respect to physiological saline of the water-absorbent resin particles is measured by the method described in the Examples section.

In view of more suitably achieving the effect of the present invention, a physiological saline retention capacity of the water-absorbent resin particles is preferably 30 g/g or more, more preferably 36 g/g or more, and still more preferably 41 g/g or more; and is preferably 70 g/g or less, more preferably 65 g/g or less, and still more preferably 60 g/g or less. Preferred ranges include from 30 to 70 g/g, from 30 to 65 g/g, from 30 to 60 g/g, from 36 to 70 g/g, from 36 to 65 g/g, from 36 to 60 g/g, from 41 to 70 g/g, from 41 to 65 g/g, and from 41 to 60 g/g.

The physiological saline retention capacity of the water-absorbent resin particles is measured by the method described in the Examples section.

In view of more suitably achieving the effect of the present invention, a physiological saline absorption capacity of the water-absorbent resin particles is preferably 45 g/g or more, 52 g/g or more, or 59 g/g or more; and is preferably 80 g/g or less, 74 g/g or less, or 69 g/g or less. Preferred ranges of the physiological saline absorption capacity of the water-absorbent resin particles include from 45 to 80 g/g, from 45 to 74 g/g, from 45 to 69 g/g, from 52 to 80 g/g, from 52 to 74 g/g, from 52 to 69 g/g, from 59 to 80 g/g, from 59 to 74 g/g, and from 59 to 69 g/g.

The physiological saline absorption capacity of the water-absorbent resin particles is measured by the method described in the Examples section.

In view of more suitably achieving the effect of the present invention, a difference between the physiological saline absorption capacity and the physiological saline retention capacity is preferably 5 or more, or 10 or more; and is preferably 30 or less, 25 or less, or 20 or less. Preferred ranges include from 5 to 30, from 5 to 25, from 5 to 20, from 10 to 30, from 10 to 25, and from 10 to 20.

In view of more suitably achieving the effect of the present invention, the water-absorbent resin particles of the present invention exhibits a re-wet amount in a curved state and under pressure, as measured in accordance with "GB/T 28004.1-2021 NATIONAL STANDARD OF THE PEOPLE'S REPUBLIC OF CHINA, Disposable diapers- Part 1: Disposable diapers for baby", of preferably 10 g or less, and more preferably 8 g or less. The lower limit is preferably 0 g. Preferred ranges include from 0 to 10 g and from 0 to 8 g.

In view of more suitably achieving the effect of the present invention, the water-absorbent resin particles of the present invention exhibits a total absorption time (a sum of a first absorption time and a second absorption time of a test liquid), as measured in accordance with "GB/T 28004.1-2021 NATIONAL STANDARD OF THE PEOPLE'S REPUBLIC OF CHINA, Disposable diapers- Part 1: Disposable diapers for baby", of preferably 90 seconds or less, more preferably 80 seconds or less, and still more preferably 75 seconds or less; or preferably 20 seconds or more. Preferred ranges include from 20 to 90 seconds, from 20 to 80 seconds, and from 20 to 75 seconds.

The re-wet amount in a curved state and under pressure and the total absorption time (the sum of the first absorption time (seconds) and second absorption time (seconds) of the test liquid), as measured in accordance with "GB/T 28004.1-2021 NATIONAL STANDARD OF THE PEOPLE'S REPUBLIC OF CHINA, Disposable diapers- Part 1: Disposable diapers for baby", are each measured by the method described in the Examples section.

The water-absorbent resin particles of the present invention are formed of a crosslinked product of a polymer of a water-soluble ethylenically unsaturated monomer, that is, a crosslinked polymer having a structural unit derived from a water-soluble ethylenically unsaturated monomer.

The water-absorbent resin particles of the present invention are in a form in which fine particles (primary particles) are agglomerated (secondary particles). Examples of the shape of the primary particles include a substantially spherical shape, an indefinite crushed shape, and a plate shape. The water-absorbent resin particles of the present invention that are the secondary particles may have various shapes. Examples of the shape of the water-absorbent resin particles include a granular shape, a substantially spherical shape, an indefinite crushed shape, a plate shape, a fibrous shape, a flake shape, or a shape formed by agglomeration of such a resin. The water-absorbent resin particles preferably have, for example, a granular shape, a substantially spherical shape, an indefinite crushed shape, a fibrous shape, or a shape formed by agglomeration of such a resin.

In view of increasing the water absorption rate (reducing the absorption time) of the absorbent material while more suitably achieving the effect of the present invention, the water-absorbent resin particles preferably have a median particle size of 200 µm or more, 250 µm or more, 300 µm or more, 320 µm or more, or 350 µm or more. From the same viewpoint, the median particle size is preferably 700 µm or less, 600 µm or less, 550 µm or less, 500 µm or less, or 450 µm or less. Preferable ranges of the median particle size include from 200 to 700 µm, from 200 to 600 µm, from 200 to 550 µm, from 200 to 500 µm, from 200 to 450 µm, from 250 to 700 µm, from 250 to 600 µm, from 250 to 550 µm, from 250 to 500 µm, from 250 to 450 µm, from 300 to 700 µm, from 300 to 600 µm, from 300 to 550 µm, from 300 to 500 µm, from 300 to 450 µm, from 350 to 700 µm, from 350 to 600 µm, from 350 to 550 µm, from 350 to 500 µm, and from 350 to 450 µm.

The median particle size of the water-absorbent resin particles can be measured using JIS standard sieves; specifically, it is the value as measured by the method described in the Examples section.

### 2. Method for Producing Water-Absorbent Resin Particles

Methods for producing the water-absorbent resin particles of the present invention are not specifically limited as long as the water-absorbent resin particles having a value of physiological saline absorption capacity under a load of 0.69 kPa after 15 minutes of 30 mL/g or more and 60 mL/g or less can be obtained. For example, a method for producing the water-absorbent resin particles of the present invention comprises the steps of polymerizing a water-soluble ethylenically unsaturated monomer to obtain polymer particles; and subjecting the polymer particles to surface-crosslinking, in the mentioned order.

As described above, examples of methods of adjusting the value of physiological saline absorption capacity under a load of 0.69 kPa after 15 minutes of the water-absorbent resin particles of the present invention include increasing the amount of surface-crosslinking agent used in the method for producing the water-absorbent resin particles; and increasing the ratio of the water-soluble ethylenically unsaturated monomer in the second-stage aqueous monomer solution to the water-soluble ethylenically unsaturated monomer in the first-stage aqueous monomer solution. These methods can strengthen the surface-crosslinked layer of the water-absorbent resin particles, so that the liquid can be suitably absorbed and retained under a low load condition of 0.69 kPa and in a short time that is the value after 15 minutes. The method for producing the water-absorbent resin particles of the present invention will be described below in detail.

### <Polymerization Step>

The polymerization step is the step of polymerizing a water-soluble ethylenically unsaturated monomer to obtain polymer particles. Examples of methods of polymerizing the water-soluble ethylenically unsaturated monomer include representative polymerization methods such as aqueous polymerization, spray droplet polymerization, emulsion polymerization, and reversed-phase suspension polymerization. In aqueous polymerization, polymerization is performed by heating a water-soluble ethylenically unsaturated monomer solution, optionally with stirring. In reversed-phase suspension polymerization, polymerization is performed by heating the water-soluble ethylenically unsaturated monomer with stirring in a hydrocarbon dispersion medium. Preferred among the above-mentioned methods is, for example, reversed-phase suspension polymerization, in view of improving general water absorption performance (in terms of physiological saline retention capacity, physiological saline absorption capacity, and the like) while setting the value of physiological saline absorption capacity under a load of 0.69 kPa after 15 minutes of the water-absorbent resin particles in the range of 30 to 60 mL/g. In the polymerization step, an internal-crosslinking agent may be optionally added to the water-soluble ethylenically unsaturated monomer to obtain crosslinked polymer particles (hydrous gel) having an internal-crosslinked structure. An example of the polymerization step will be hereinafter described.

### [Water-Soluble Ethylenically Unsaturated Monomer]

Examples of the water-soluble ethylenically unsaturated monomer include (meth)acrylic acid ("acrylic" and "methacrylic" are herein collectively referred to as "(meth)acrylic"; the same applies below) and salts thereof; 2-(meth)acrylamido-2-methylpropanesulfonic acid and salts thereof; nonionic monomers, such as (meth)acrylamide, N,N-dimethyl(meth)acrylamide, 2-hydroxyethyl (meth)acrylate, N-methylol(meth)acrylamide, and polyethylene glycol mono(meth)acrylate; and amino group-containing unsaturated monomers, such as N,N-diethylaminoethyl (meth)acrylate, N,N-diethylaminopropyl (meth)acrylate, and diethylaminopropyl (meth)acrylamide, as well as quaternary compounds thereof. Preferred among these water-soluble ethylenically unsaturated monomers are (meth)acrylic acid and salts thereof, (meth)acrylamide, and N,N-dimethylacrylamide, and more preferred are (meth)acrylic acid and salts thereof, because they are readily industrially available, for example. These water-soluble ethylenically unsaturated monomers may be used alone or in combinations of two or more.

Among these water-soluble ethylenically unsaturated monomers, acrylic acid and salts thereof are widely used as raw materials of water-absorbent resin particles. Copolymers of acrylic acid and/or salts thereof with other water-soluble ethylenically unsaturated monomers as mentioned above may also be used. In this case, acrylic acid and/or a salt thereof as a main water-soluble ethylenically unsaturated monomer is preferably used in an amount of 70 to 100 mol% based on the total amount of water-soluble ethylenically unsaturated monomers.

The water-soluble ethylenically unsaturated monomer as an aqueous solution may be dispersed in a hydrocarbon dispersion medium and then subjected to reversed-phase suspension polymerization. When the water-soluble ethylenically unsaturated monomer is in the form of an aqueous solution, the dispersion efficiency in the hydrocarbon dispersion medium can be increased. The concentration of the water-soluble ethylenically unsaturated monomer in the aqueous solution is preferably in the range of 20% by mass to not more than the saturated concentration. The concentration of the water-soluble ethylenically unsaturated monomer is more preferably 55% by mass or less, still more preferably 50% by mass or less, and even more preferably 45% by mass or less. On the other hand, the concentration of the water-soluble ethylenically unsaturated monomer is more preferably 25% by mass or more, still more preferably 28% by mass or more, and even more preferably 30% by mass or more.

The ratio of the water-soluble ethylenically unsaturated monomer in the second-stage aqueous monomer solution to the water-soluble ethylenically unsaturated monomer in the first-stage aqueous monomer solution (second-stage water-soluble ethylenically unsaturated monomer/first-stage water-soluble ethylenically unsaturated monomer) is preferably 0.1 to 3.0, more preferably 0.5 to 2.5, still more preferably 1.0 to 2.0, and even more preferably 1.5 to 1.8, in view of adjusting the value of physiological saline absorption capacity under a load of 0.69 kPa after 15 minutes in the preferred ranges.

When the water-soluble ethylenically unsaturated monomer has an acid group such as (meth)acrylic acid or 2-(meth)acrylamido-2-methylpropanesulfonic acid, the acid group may be optionally neutralized with an alkaline neutralizing agent, before the water-soluble ethylenically unsaturated monomer is used. Examples of such alkaline neutralizing agents include alkali metal salts, such as sodium hydroxide, sodium carbonate, sodium hydrogen carbonate, potassium hydroxide, and potassium carbonate; and ammonia. These alkaline neutralizing agents may be used in the form of an aqueous solution to facilitate the neutralization process. The above-mentioned alkaline neutralizing agents may be used alone or in combinations of two or more.

The neutralization degree of the water-soluble ethylenically unsaturated monomer with an alkaline neutralizing agent, calculated as the neutralization degree of all acid groups in the water-soluble ethylenically unsaturated monomer, is preferably 40 to 100 mol%, more preferably 50 to 90 mol%, still more preferably 60 to 85 mol%, and even more preferably 70 to 80 mol%.

### [Radical Polymerization Initiator]

Examples of the radical polymerization initiator added in the polymerization step include persulfates, such as potassium persulfate, ammonium persulfate, and sodium persulfate; peroxides, such as methyl ethyl ketone peroxide, methyl isobutyl ketone peroxide, di-t-butyl peroxide, t-butyl cumyl peroxide, t-butyl peroxyacetate, t-butyl peroxyisobutyrate, t-butyl peroxypivalate, and hydrogen peroxide; and azo compounds, such as 2,2'-azobis(2-amidinopropane) dihydrochloride, 2,2'-azobis[2-(N-phenylamidino)propane] dihydrochloride, 2,2'-azobis[2-(N-allylamidino)propane] dihydrochloride, 2,2'-azobis{2-[1-(2-hydroxyethyl)-2-imidazolin-2-yl]propane} dihydrochloride, 2,2'-azobis{2-methyl-N-[1,1-bis(hydroxymethyl)-2-hydroxyethyl]propionamide}, 2,2'-azobis[2-methyl-N-(2-hydroxyethyl)-propionamide], and 4,4'-azobis(4-cyanovaleric acid). Preferred among these radical polymerization initiators are potassium persulfate, ammonium persulfate, sodium persulfate, and 2,2'-azobis(2-amidinopropane) dihydrochloride, because they are readily available and easy to handle. These radical polymerization initiators may be used alone or in combinations of two or more. The above-mentioned radical polymerization initiators may also be used in combination with a reducing agent, such as sodium sulfite, sodium hydrogensulfite, ferrous sulfate, or L-ascorbic acid, and used as a redox polymerization initiator.

The amount of the radical polymerization initiator used is, for example, 0.00005 to 0.01 mol per mole of the water-soluble ethylenically unsaturated monomer. When the radical polymerization initiator is used in the above-defined range of amounts, the occurrence of an abrupt polymerization reaction can be avoided, and the polymerization reaction can be completed in an appropriate period of time.

### [Internal-Crosslinking Agent]

Examples of the internal-crosslinking agent include those that can crosslink the polymer of the water-soluble ethylenically unsaturated monomer to be used, for example: unsaturated polyesters obtained by reacting polyols, such as diols and triols, e.g., (poly)ethylene glycol ["(poly)" means both cases with and without the prefix "poly"; the same applies below], (poly)propylene glycol, 1,4-butanediol, 1,6-hexanediol, trimethylolpropane, and (poly)glycerin, with unsaturated acids, such as (meth)acrylic acid, maleic acid, and fumaric acid; bisacrylamides, such as N,N-methylenebisacrylamide; di or tri(meth)acrylic acid esters obtained by reacting polyepoxides with (meth)acrylic acid; carbamyl di(meth)acrylates obtained by reacting polyisocyanates, such as tolylene diisocyanate and hexamethylene diisocyanate, with hydroxyethyl (meth)acrylate; compounds having two or more polymerizable unsaturated groups, such as allylated starch, allylated cellulose, diallyl phthalate, N,N',N"-triallyl isocyanurate, and divinylbenzene; polyglycidyl compounds, such as diglycidyl compounds, e.g., (poly)ethylene glycol diglycidyl ether, (poly)propylene glycol diglycidyl ether, and (poly)glycerin diglycidyl ether, and triglycidyl compounds; epihalohydrin compounds, such as epichlorohydrin, epibromohydrin, and α-methylepichlorohydrin; compounds having two or more reactive functional groups, such as isocyanate compounds, e.g., 2,4-tolylene diisocyanate and hexamethylene diisocyanate; and oxetane compounds, such as 3-methyl-3-oxetanemethanol, 3-ethyl-3-oxetanemethanol, 3-butyl-3-oxetanemethanol, 3-methyl-3-oxetaneethanol, 3-ethyl-3-oxetaneethanol, and 3-butyl-3-oxetaneethanol. Among these internal-crosslinking agents, polyglycidyl compounds are preferably used, diglycidyl ether compounds are more preferably used, and (poly)ethylene glycol diglycidyl ether, (poly)propylene glycol diglycidyl ether, and (poly)glycerin diglycidyl ether are still more preferably used. These internal-crosslinking agents may be used alone or in combinations of two or more.

The amount of the internal-crosslinking agent used in the first-stage aqueous monomer solution is preferably 0.000001 to 0.005 mol, more preferably 0.00001 to 0.002 mol, still more preferably 0.00001 to 0.001 mol, and even more preferably 0.00005 to 0.0005 mol, per mole of the water-soluble ethylenically unsaturated monomer.

The amount of the internal-crosslinking agent used in the second-stage aqueous monomer solution is preferably 0 to 0.005 mol, more preferably 0 to 0.0005 mol, still more preferably 0 to 0.0001 mol, and particularly preferably 0 to 0.00004 mol, per mole of the water-soluble ethylenically unsaturated monomer, in view of improving general water absorption performance (in terms of physiological saline retention capacity, physiological saline absorption capacity, and the like).

### [Hydrocarbon Dispersion Medium]

Examples of the hydrocarbon dispersion medium include C₆₋₈ aliphatic hydrocarbons, such as n-hexane, n-heptane, 2-methylhexane, 3-methylhexane, 2,3-dimethylpentane, 3-ethylpentane, and n-octane; alicyclic hydrocarbons, such as cyclohexane, methylcyclohexane, cyclopentane, methylcyclopentane, trans-1,2-dimethylcyclopentane, cis-1,3-dimethylcyclopentane, and trans-1,3-dimethylcyclopentane; and aromatic hydrocarbons, such as benzene, toluene, and xylene. Among these hydrocarbon dispersion media, n-hexane, n-heptane, and cyclohexane, which are readily industrially available, stable in quality, and inexpensive, are particularly suitably used. These hydrocarbon dispersion media may be used alone or in combinations of two or more. Favorable results are also obtained using a commercially available mixture of hydrocarbon dispersion media, such as Exxsol Heptane (manufactured by Exxon Mobil Corporation; containing 75 to 85% by mass of heptane and its isomeric hydrocarbons).

The amount of the hydrocarbon dispersion medium used is preferably 100 to 1500 parts by mass, and more preferably 200 to 1400 parts by mass, per 100 parts by mass of the first-stage water-soluble ethylenically unsaturated monomer, in view of homogeneously dispersing the water-soluble ethylenically unsaturated monomer, and facilitating control of the polymerization temperature. As described below, reversed-phase suspension polymerization is performed in one stage (single stage) or two or more multiple stages. The above-mentioned first-stage polymerization refers to the first-stage polymerization reaction in single-stage polymerization or multi-stage polymerization (the same applies below).

### [Dispersion Stabilizer]

### (Surfactant)

In reversed-phase suspension polymerization, a dispersion stabilizer may be used to improve the dispersion stability of the water-soluble ethylenically unsaturated monomer in the hydrocarbon dispersion medium. A surfactant may be used as such a dispersion stabilizer.

Examples of usable surfactants include sucrose fatty acid esters, polyglycerol fatty acid esters, sorbitan fatty acid esters, polyoxyethylene sorbitan fatty acid esters, polyoxyethylene glycerin fatty acid esters, sorbitol fatty acid esters, polyoxyethylene sorbitol fatty acid esters, polyoxyethylene alkyl ethers, polyoxyethylene alkyl phenyl ethers, polyoxyethylene castor oil, polyoxyethylene hydrogenated castor oil, alkyl allyl formaldehyde condensed polyoxyethylene ethers, polyoxyethylene-polyoxypropylene block copolymers, polyoxyethylene polyoxypropyl alkyl ethers, polyethylene glycol fatty acid esters, alkyl glucosides, N-alkyl glyconamides, polyoxyethylene fatty acid amides, polyoxyethylene alkylamines, polyoxyethylene alkyl ether phosphates, and polyoxyethylene alkyl allyl ether phosphates. Among these surfactants, sorbitan fatty acid esters, polyglycerol fatty acid esters, and sucrose fatty acid esters are particularly preferably used, in view of dispersion stability of the monomer. These surfactants may be used alone or in combinations of two or more.

The amount of the surfactant used is preferably 0.1 to 30 parts by mass, and more preferably 0.3 to 20 parts by mass, per 100 parts by mass of the first-stage water-soluble ethylenically unsaturated monomer.

### (Polymeric Dispersion Agent)

A polymeric dispersion agent may be used in combination with the above-described surfactant, as the dispersion stabilizer to be used in reversed-phase suspension polymerization.

Examples of the polymeric dispersion agent include maleic anhydride modified polyethylene, maleic anhydride modified polypropylene, maleic anhydride modified ethylene-propylene copolymers, maleic anhydride modified EPDM (ethylene-propylene-diene terpolymers), maleic anhydride modified polybutadiene, maleic anhydride-ethylene copolymers, maleic anhydride-propylene copolymers, maleic anhydride-ethylene-propylene copolymers, maleic anhydride-butadiene copolymers, polyethylene, polypropylene, ethylene-propylene copolymers, oxidized polyethylene, oxidized polypropylene, oxidized ethylene-propylene copolymers, ethylene-acrylic acid copolymers, ethyl cellulose, and ethyl hydroxyethyl cellulose. Among these polymeric dispersion agents, maleic anhydride modified polyethylene, maleic anhydride modified polypropylene, maleic anhydride modified ethylene-propylene copolymers, maleic anhydride-ethylene copolymers, maleic anhydride-propylene copolymers, maleic anhydride-ethylene-propylene copolymers, polyethylene, polypropylene, ethylene-propylene copolymers, oxidized polyethylene, oxidized polypropylene, and oxidized ethylene-propylene copolymers are particularly preferably used, in view of dispersion stability of the monomer. These polymeric dispersion agents may be used alone or in combinations of two or more.

The amount of the polymeric dispersion agent used is preferably 0.1 to 30 parts by mass, and more preferably 0.3 to 20 parts by mass, per 100 parts by mass of the first-stage water-soluble ethylenically unsaturated monomer.

### [Other Components]

In the method for producing the water-absorbent resin particles, other components may be optionally added to the aqueous solution containing the water-soluble ethylenically unsaturated monomer, which is then subjected to reversed-phase suspension polymerization. Various additives such as thickeners and chain transfer agents can be added as the other components.

For example, a thickener may be added to the aqueous solution containing the water-soluble ethylenically unsaturated monomer, which is then subjected to reversed-phase suspension polymerization. By thus adding a thickener to adjust the viscosity of the aqueous solution, the median particle size obtained by reversed-phase suspension polymerization can be controlled.

Examples of usable thickeners include hydroxyethylcellulose, hydroxypropylcellulose, methylcellulose, carboxymethylcellulose, polyacrylic acid, (partially) neutralized polyacrylic acid, polyethylene glycol, polyacrylamide, polyethyleneimine, dextrin, sodium alginate, polyvinyl alcohol, polyvinylpyrrolidone, and polyethylene oxide. When the stirring rate during the polymerization is the same, the higher the viscosity of the water-soluble ethylenically unsaturated monomer solution, the larger the primary particles and/or secondary particles of the resulting particles tend to be.

### [Reversed-Phase Suspension Polymerization]

Reversed-phase suspension polymerization includes, for example, dispersing an aqueous monomer solution containing the water-soluble ethylenically unsaturated monomer in a hydrocarbon dispersion medium, in the presence of a dispersion stabilizer. Here, as long as the dispersion stabilizer (a surfactant and a polymeric dispersion agent) is added before the beginning of the polymerization reaction, it may be added either before or after the addition of the aqueous monomer solution.

In particular, in view of readily reducing the amount of the hydrocarbon dispersion medium remaining in the resulting water-absorbent resin particles, it is preferred to disperse the aqueous monomer solution in the hydrocarbon dispersion medium in which a polymeric dispersion agent is dispersed, and then disperse a surfactant in the resulting dispersion, followed by polymerization.

Reversed-phase suspension polymerization as described above can be performed in one stage or two or more multiple stages. In view of improving productivity, reversed-phase suspension polymerization is preferably performed in two or three stages.

Reversed-phase suspension polymerization in two or more multiple stages may be performed as follows: the first stage of reversed-phase suspension polymerization is performed; subsequently, the water-soluble ethylenically unsaturated monomer is added to and mixed with the reaction mixture obtained by the first-stage polymerization reaction, and the second and subsequent stages of reversed-phase suspension polymerization are performed in the same manner as in the first stage. In each of the second and subsequent stages of reversed-phase suspension polymerization, it is preferred to add, in addition to the water-soluble ethylenically unsaturated monomer, a radical polymerization initiator within the above-described range of molar ratios of each component relative to the water-soluble ethylenically unsaturated monomer, based on the amount of the water-soluble ethylenically unsaturated monomer added in each of the second and subsequent stages of reversed-phase suspension polymerization, and then perform reversed-phase suspension polymerization. In the second and subsequent stages of polymerization, an internal-crosslinking agent may also be optionally added to the water-soluble ethylenically unsaturated monomer.

The reaction temperature during the polymerization reaction is preferably 20 to 110°C, and more preferably 40 to 90°C, in view of allowing the polymerization to proceed quickly to reduce the polymerization time for improved economic efficiency, and readily removing the heat of polymerization to perform the reaction smoothly.

### <Dehydration Step>

After reversed-phase suspension polymerization is performed as described above, the method may include a dehydration step of removing the water, the hydrocarbon dispersion medium, and the like by distillation, by adding external energy, such as heat. To remove the water in the hydrous gel after reversed-phase suspension polymerization, the system in which the hydrous gel is dispersed in the hydrocarbon dispersion medium is heated to distill the water and the hydrocarbon dispersion medium out of the system by azeotropic distillation. Here, if the distilled hydrocarbon dispersion medium only is returned into the system, continuous azeotropic distillation can be performed. This is preferable in view of preventing deterioration of the resin, because the temperature within the system during drying is maintained at a temperature not higher than the azeotropic temperature with the hydrocarbon dispersion medium. By controlling the treatment conditions for the dehydration step after the polymerization to adjust the amount of water to be removed (that is, adjust the water content in the polymer particles), various kinds of performance of the resulting water-absorbent resin particles can be controlled.

In the dehydration step, the dehydration treatment by distillation may be performed under atmospheric pressure. When the dehydration treatment is performed under atmospheric pressure, the dehydration temperature is preferably 70 to 250°C, more preferably 80 to 180°C, still more preferably 80 to 140°C, and even more preferably 90 to 130°C.

### <Surface-Crosslinking Step>

The surface-crosslinking step is the step of subjecting the polymer particles obtained in the polymerization step to surface-crosslinking. When the polymer particles are crosslinked polymer particles (hydrous gel), the surface-crosslinking step is the step of crosslinking the hydrous gel having an internal-crosslinked structure obtained by polymerization of the water-soluble ethylenically unsaturated monomer, by adding a surface-crosslinking agent thereto (surface-crosslinking reaction). The surface-crosslinking reaction is preferably performed in the presence of a surface-crosslinking agent, after the polymerization of the water-soluble ethylenically unsaturated monomer. By thus subjecting the hydrous gel having an internal-crosslinked structure to the surface-crosslinking reaction after the polymerization, so that the crosslinking density near the surfaces of the water-absorbent resin particles falls in a specific range, it is possible to obtain water-absorbent resin particles that exhibit improvement in various kinds of performance, such as water absorption capacity under load.

Examples of the surface-crosslinking agent include compounds with two or more reactive functional groups. Examples include polyols, such as ethylene glycol, propylene glycol, 1,4-butanediol, diethylene glycol, triethylene glycol, trimethylolpropane, glycerin, polyoxyethylene glycol, polyoxypropylene glycol, and polyglycerin; polyglycidyl compounds, such as (poly)ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether; haloepoxy compounds, such as epichlorohydrin, epibromohydrin, and α-methyl epichlorohydrin; isocyanate compounds, such as 2,4-tolylene diisocyanate and hexamethylene diisocyanate; oxetane compounds, such as 3-methyl-3-oxetanemethanol, 3-ethyl-3-oxetanemethanol, 3-butyl-3-oxetanemethanol, 3-methyl-3-oxetaneethanol, 3-ethyl-3-oxetaneethanol, and 3-butyl-3-oxetaneethanol; oxazoline compounds, such as 1,2-ethylenebisoxazoline; carbonate compounds (e.g., alkylene carbonates), such as ethylene carbonate, propylene carbonate, 4,5-dimethyl-1,3-dioxolan-2-one, 4,4-dimethyl-1,3-dioxolan-2-one, 4-ethyl-1,3-dioxolan-2-one, 4-hydroxymethyl-1,3-dioxolan-2-one, 1,3-dioxan-2-one, 4-methyl-1,3-dioxan-2-one, 4,6-dimethyl-1,3-dioxan-2-one, and 1,3-dioxolan-2-one; and hydroxyalkylamide compounds, such as bis[N,N-di(β-hydroxyethyl)]adipamide. Preferred among these surface-crosslinking agents are polyglycidyl compounds, such as (poly)ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether. These surface-crosslinking agents may be used alone or in combinations of two or more.

In view of adjusting the value of physiological saline absorption capacity under a load of 0.69 kPa after 15 minutes in the preferred ranges, the amount of the surface-crosslinking agent used is preferably 0.00001 to 0.01 mol, more preferably 0.00005 to 0.005 mol, still more preferably 0.0001 to 0.001 mol, and even more preferably 0.0004 to 0.0009 mol, per mole of the total amount of the water-soluble ethylenically unsaturated monomer used for polymerization.

The surface-crosslinking agent may be added as is or as an aqueous solution. Optionally, a solution of the surface-crosslinking agent in a hydrophilic organic solvent may be added. Examples of the hydrophilic organic solvent include lower alcohols, such as methyl alcohol, ethyl alcohol, n-propyl alcohol, and isopropyl alcohol; ketones, such as acetone and methyl ethyl ketone; ethers, such as diethyl ether, dioxane, and tetrahydrofuran; amides, such as N,N-dimethylformamide; and sulfoxides, such as dimethylsulfoxide. These hydrophilic organic solvents may be used alone, in combinations of two or more, or as a solvent mixture with water.

The surface-crosslinking agent may be added at any time after the polymerization reaction of the water-soluble ethylenically unsaturated monomer is substantially completed. The surface-crosslinking agent is preferably added in the presence of 1 to 400 parts by mass of water, more preferably 5 to 200 parts by mass of water, still more preferably 10 to 100 parts by mass of water, even more preferably 15 to 60 parts by mass of water, per 100 parts by mass of the water-soluble ethylenically unsaturated monomer used for polymerization. The amount of water herein refers to the total amount of the water contained in the reaction system and the water optionally used during the addition of the surface-crosslinking agent.

The reaction temperature during the surface-crosslinking reaction is preferably 50 to 250°C, more preferably 60 to 180°C, still more preferably 60 to 140°C, and even more preferably 70 to 120°C. The reaction time of the surface-crosslinking reaction is preferably 1 to 300 minutes, and more preferably 5 to 200 minutes.

### <Drying Step>

After the surface-crosslinking is performed as described above, the method may include a drying step of removing the water, the hydrocarbon dispersion medium, and the like by distillation, by adding external energy, such as heat. The polymer particles after the surface-crosslinking are dried to distill off the water and hydrocarbon dispersion medium to obtain water-absorbent resin particles.

In the drying step, the drying treatment by distillation may be performed under atmospheric pressure or reduced pressure. The drying treatment may also be performed in a stream of nitrogen or the like, in view of improving the drying efficiency. When the drying treatment is performed under atmospheric pressure, the drying temperature is preferably 70 to 250°C, more preferably 80 to 180°C, still more preferably 80 to 140°C, and even more preferably 90 to 130°C. When the drying treatment is performed under reduced pressure, the drying temperature is preferably 40 to 160°C, and more preferably 50 to 110°C.

When the surface-crosslinking step with a surface-crosslinking agent is performed after the polymerization of the monomer by reversed-phase suspension polymerization, the drying step by distillation is performed as described above, after the completion of the surface-crosslinking step. Alternatively, the surface-crosslinking step and the drying step may be performed simultaneously.

The water-absorbent resin particles of the present invention may contain additives suitable for its purpose. Examples of such additives include inorganic powders, surfactants, oxidizing agents, reducing agents, metal chelating agents, radical chain inhibitors, antioxidants, and anti-bacterial agents. For example, the flowability of the water-absorbent resin particles can be further improved by adding 0.05 to 5 parts by mass of amorphous silica as an inorganic powder to 100 parts by mass of the water-absorbent resin particles. The additives are preferably hydrophilic or water-soluble.

### 3. Absorbent Material and Absorbent Article

The water-absorbent resin particles of the present invention constitute an absorbent material used for hygienic materials, such as sanitary articles and disposable diapers, for example, and are suitably used for an absorbent article including the absorbent material.

The absorbent material of the present invention contains the water-absorbent resin particles of the present invention. The absorbent material may further contain hydrophilic fibers. Examples of the structure of the absorbent material include a sheet-like structure in which the water-absorbent resin particles are fixed on a nonwoven fabric or between a plurality of nonwoven fabrics; a mixed dispersion obtained by mixing the water-absorbent resin particles and hydrophilic fibers to give a homogeneous composition; a sandwich structure in which the water-absorbent resin particles are sandwiched between layered hydrophilic fibers; and a structure in which the water-absorbent resin particles and hydrophilic fibers are wrapped in a tissue. The absorbent material may also contain other components, for example, adhesive binders such as thermal bonding synthetic fibers, hot melt adhesives, and adhesive emulsions, for improving the shape retention properties of the absorbent material.

The water-absorbent resin particles in the absorbent material of the present invention have a basis weight of 30 g/m² or more and 500 g/m² or less. The basis weight is preferably 100 g/m² or more and 400 g/m² or less.

The water-absorbent resin particles in the mixed dispersion of the present invention have a basis weight of 30 g/m² or more and 500 g/m² or less. The basis weight is preferably 100 g/m² or more, more preferably 120 g/m² or more, and still more preferably 140 g/m² or more; and is preferably 300 g/m² or less, more preferably 250 g/m² or less, and still more preferably 200 g/m² or less.

The water-absorbent resin particles in the sheet-like structure of the present invention have a basis weight of 150 g/m² or more and 500 g/m² or less. The basis weight is preferably 250 g/m² or more, more preferably 300 g/m² or more, and still more preferably 350 g/m² or more; and is preferably 450 g/m² or less, more preferably 400 g/m² or less, and still more preferably 350 g/m² or less.

The hydrophilic fibers may be, for example, at least one selected from the group consisting of finely ground wood pulp, cotton, cotton linter, rayon, cellulose acetate, polyamides, polyesters, and polyolefins. Examples include cellulose fibers, such as cotton-like pulp made from wood, mechanical pulp, chemical pulp, and semi-chemical pulp; artificial cellulose fibers, such as rayon and acetate; and fibers made of synthetic resins, such as hydrophilized polyamides, polyesters, and polyolefins. The hydrophilic fibers typically have an average fiber length of 0.1 to 10 mm. Alternatively, the average fiber length may be 0.5 to 5 mm.

The hydrophilic fibers in the absorbent material of the present invention have a basis weight of 0 g/m² or more and 800 g/m² or less. The basis weight is preferably 0 g/m² or more and 500 g/m² or less.

The hydrophilic fibers in the mixed dispersion of the present invention have a basis weight of 50 g/m² or more and 800 g/m² or less. The basis weight is preferably 100 g/m² or more, more preferably 120 g/m² or more, and still more preferably 140 g/m² or more; and is preferably 700 g/m² or less, more preferably 600 g/m² or less, and still more preferably 500 g/m² or less.

The hydrophilic fibers in the sheet-like structure of the present invention have a basis weight of 0 g/m² or more and 100 g/m² or less. The basis weight is preferably 0 g/m² or more and 80 g/m² or less, more preferably 0 g/m² or more and 50 g/m² or less, and still more preferably 0 g/m².

The content of the water-absorbent resin particles in the absorbent material is preferably 5 to 100% by mass, more preferably 10 to 95% by mass, still more preferably 20 to 90% by mass, and even more preferably 30 to 80% by mass.

It is possible to obtain the absorbent article of the present invention by holding the absorbent material obtained using the water-absorbent resin particles of the present invention between a liquid-permeable sheet (top sheet) that allows a liquid to pass through and a liquid-impermeable sheet (back sheet) that does not allow a liquid to pass through. The liquid-permeable sheet is positioned on the side that is brought into contact with the body, and the liquid-impermeable sheet is positioned opposite to the side that is brought into contact with the body.

Examples of the liquid-permeable sheet include porous synthetic resin sheets and nonwoven fabrics of the types such as air-through type, spunbond type, chemical bond type, and needle punch type, which are made of fibers of polyethylene, polypropylene, polyester, and the like. Examples of the liquid-impermeable sheet include synthetic resin films made of resins such as polyethylene, polypropylene, and polyvinyl chloride. The liquid-permeable sheet is preferably at least one selected from the group consisting of a thermally bonded nonwoven fabric, an air-through nonwoven fabric, a spunbond nonwoven fabric, and a spunbond/melt-blown/spunbond nonwoven fabric.

The liquid-permeable sheet has a basis weight of preferably 5 g/m² or more and 100 g/m² or less, more preferably 10 g/m² or more and 60 g/m² or less. The surface of the liquid-permeable sheet may be embossed or perforated in order to improve the liquid diffusibility. The embossing and perforating can be performed by known methods.

Examples of the liquid-impermeable sheet include a sheet made of a synthetic resin such as polyethylene, polypropylene, or polyvinyl chloride, a sheet made of a nonwoven fabric such as a spunbond/melt-blown/spunbond (SMS) nonwoven fabric in which a water-resistant melt-blown nonwoven fabric is sandwiched between high-strength spunbond nonwoven fabrics, and a sheet made of a composite material of these synthetic resins and a nonwoven fabric (for example, a spunbond nonwoven fabric or a spunlace nonwoven fabric). As the liquid-impermeable sheet, a sheet made of a synthetic resin mainly containing a low-density polyethylene (LDPE) resin can also be used. The liquid-impermeable sheet may be, for example, a sheet made of a synthetic resin having a basis weight of 10 to 50 g/m².

The absorbent article preferably includes a laminate having an absorbent material containing the water-absorbent resin particles and core wraps sandwiching upper and lower portions of the absorbent material; a liquid-permeable sheet disposed on an upper surface of the laminate; and a liquid-impermeable sheet disposed on a surface of the laminate opposite to the liquid-permeable sheet side.

### 4. Additional Matters

The present specification includes at least the inventions as set forth in (1) to (8) below.
(1) Water-absorbent resin particles having a value of physiological saline absorption capacity under a load of 0.69 kPa after 15 minutes of 30 to 60 mL/g, 30 to 58 mL/g, 30 to 55 mL/g, 32 to 60 mL/g, 32 to 58 mL/g, 32 to 55 mL/, 34 to 60 mL/g, 34 to 58 mL/g, 34 to 55 mL/g, 35 to 60 mL/g, 35 to 58 mL/g, or 35 to 55 mL/g.
(2) The water-absorbent resin particles according to (1) above, wherein the water-absorbent resin particles have a value of non-pressurization DW after 3 minutes of 20 to 50 mL/g, 20 to 48 mL/g, 20 to 46 mL/g, 30 to 50 mL/g, 30 to 48 mL/g, 30 to 46 mL/g, 33 to 50 mL/g, 33 to 48 mL/g, 33 to 46 mL/g, 36 to 50 mL/g, 36 to 48 mL/g, 36 to 46 mL/g, 39 to 50 mL/g, 39 to 48 mL/g, or 39 to 46 mL/g.
(3) The water-absorbent resin particles according to (1) or (2) above, wherein the water-absorbent resin particles have a water absorption rate with respect to physiological saline of 60 seconds or less, 20 to 60 seconds, 20 to 55 seconds, 20 to 50 seconds, 25 to 60 seconds, 25 to 55 seconds, 25 to 50 seconds, 30 to 60 seconds, 30 to 55 seconds, or 30 to 50 seconds.
(4) The water-absorbent resin particles according to any one of (1) to (3) above, wherein the water-absorbent resin particles have a physiological saline retention capacity of 30 g/g or more, 30 to 70 g/g, 30 to 65 g/g, 30 to 60 g/g, 36 to 70 g/g, 36 to 65 g/g, 36 to 60 g/g, 41 to 70 g/g, 41 to 65 g/g, or 41 to 60 g/g.
(5) The water-absorbent resin particles according to any one of (1) to (4) above, wherein the water-absorbent resin particles have a value of non-pressurization DW after 10 minutes of 30 to 80 mL/g, 30 to 75 mL/g, 30 to 72 mL/g, 45 to 80 mL/g, 45 to 75 mL/g, 45 to 72 mL/g, 50 to 80 mL/g, 50 to 75 mL/g, 50 to 72 mL/g, 54 to 80 mL/g, 54 to 75 mL/g, 54 to 72 mL/g, 56 to 80 mL/g, 56 to 75 mL/g, or 56 to 72 mL/g.
(6) The water-absorbent resin particles according to any one of (1) to (5) above, wherein the water-absorbent resin particles have a physiological saline absorption capacity of 30 g/g or more, 45 to 80 g/g, 45 to 74 g/g, 45 to 69 g/g, 52 to 80 g/g, 52 to 74 g/g, 52 to 69 g/g, 59 to 80 g/g, 59 to 74 g/g, or 59 to 69 g/g.
(7) The water-absorbent resin particles according to any one of (1) to (6) above, wherein the water-absorbent resin particles have a difference between the physiological saline absorption capacity and the physiological saline retention capacity of 5 to 30, 5 to 25, 5 to 20, 10 to 30, 10 to 25, or 10 to 20.
(8) The water-absorbent resin particles according to any one of (1) to (7) above, wherein the water-absorbent resin particles have a median particle size of 200 to 700 µm, 200 to 600 µm, 200 to 550 µm, 200 to 500 µm, 200 to 450 µm, 250 to 700 µm, 250 to 600 µm, 250 to 550 µm, 250 to 500 µm, 250 to 450 µm, 300 to 700 µm, 300 to 600 µm, 300 to 550 µm, 300 to 500 µm, 300 to 450 µm, 350 to 700 µm, 350 to 600 µm, 350 to 550 µm, 350 to 500 µm, or 350 to 450 µm.

### Examples

The present invention will be hereinafter described in detail with reference to examples and comparative examples. However, the present invention is not limited to the examples. Unless otherwise indicated, the measurement was performed in an environment at a temperature of 25 ± 2°C and a humidity of 50 ± 10%.

### <Production of Water-Absorbent Resin Particles>

### <Example 1>

### [First-Stage Polymerization Step]

An 11 cm inner diameter, 2 L volume, cylindrical round-bottomed separable flask, which was equipped with a reflux condenser, a dropping funnel, a nitrogen gas inlet tube, and an agitator with stirring blades having two stages of four inclined paddle blades with a blade diameter of 5 cm, was prepared. As a hydrocarbon dispersion medium, 292 g of n-heptane was placed in this flask, and 0.782 g of a maleic anhydride-modified ethylene-propylene copolymer (Hi-Wax 1105A from Mitsui Chemicals, Inc.) was added as a polymeric dispersion agent. The mixture was heated with stirring to 80°C to dissolve the dispersion agent and then cooled to 50°C.

In a 300 mL inner volume beaker, 92.0 g (1.03 mol) of an 80.5% by mass aqueous solution of acrylic acid was placed as a water-soluble ethylenically unsaturated monomer, and 110.1 g of a 28.0% by mass aqueous solution of sodium hydroxide was added dropwise, with external cooling, to achieve 75 mol% neutralization. Then, 0.092 g (0.339 mmol) of 2,2'-azobis(2-amidinopropane) dihydrochloride and 0.028 g (0.102 mmol) of potassium persulfate as water-soluble radical polymerization initiators, 0.0101 g (0.058 mmol) of ethylene glycol diglycidyl ether as an internal-crosslinking agent, and 37.2 g of ion exchange water were added and dissolved to prepare a first-stage aqueous monomer solution.

Then, the aqueous solution prepared as above was added to the separable flask and stirred for 10 minutes. Then, a surfactant solution prepared by heating and dissolving 0.828 g of sucrose stearate having an HLB of 3 (Ryoto sugar ester S-370 from Mitsubishi-Kagaku Foods Corporation) as a surfactant in 7.45 g of n-heptane was further added. The system was sufficiently purged with nitrogen while stirring with the agitator at a rotation speed of 500 rpm. Then, the flask was immersed in a water bath at 70°C and heated, and polymerization was performed for 60 minutes to give a first-stage polymerization slurry containing primary particles.

### [Second-Stage Polymerization Step]

In a 500 mL inner volume beaker, 128.8 g (1.44 mol) of an 80.5% by mass aqueous solution of acrylic acid was placed as a water-soluble ethylenically unsaturated monomer, and 154.2 g of a 28.0% by mass aqueous solution of sodium hydroxide was added dropwise, with external cooling, to achieve 75 mol% neutralization. Then, 0.064 g (0.237 mmol) of 2,2'-azobis(2-amidinopropane) dihydrochloride and 0.039 g (0.143 mmol) of potassium persulfate as water-soluble radical polymerization initiators, and 6.49 g of ion exchange water were added and dissolved to prepare a second-stage aqueous monomer solution.

The separable flask system was cooled to 25°C while stirring with the agitator at a rotation speed of 1000 rpm. Then, the entire amount of the second-stage aqueous solution was added to the first-stage polymerization slurry, and the system was purged with nitrogen for 30 minutes. Then, the flask was again immersed in a water bath at 70°C and heated, and the polymerization reaction was performed for 60 minutes. A hydrous gel after the second-stage polymerization was obtained by the procedure described above.

### [Surface-Crosslinking]

The flask was then immersed in an oil bath set at 125°C to remove 176.6 g of water out of the system by azeotropic distillation of the water and n-heptane, while refluxing the n-heptane. Then, 4.91 g of a 4.5% by mass aqueous solution of diethylenetriaminepentaacetic acid pentasodium salt and 2.58 g of a 3% by mass aqueous solution of sodium sulfite were added to the flask with stirring to give a second-stage polymerization slurry containing secondary particles.

The flask was then again immersed in an oil bath set at 125°C to further remove 99.5 g (276.1 g in total) of water out of the system by azeotropic distillation of the water and n-heptane, while refluxing the n-heptane. Then, 16.6 g (1.90 mmol) of a 2% by mass aqueous solution of ethylene glycol diglycidyl ether as a surface-crosslinking agent was added to the flask, and the mixture was maintained at 83°C for 2 hours.

Then, the mixture was dried by evaporating the n-heptane at 125°C to give a dried product. The dried product was passed through a sieve with a mesh size of 850 µm, and the resulting dried product was mixed with 0.2% by mass of amorphous silica (Tokusil NP-S from Oriental Silicas Corporation) to give 210.4 g of water-absorbent resin particles of Example 1.

### <Example 2>

218.6 g of water-absorbent resin particles of Example 2 were obtained as in Example 1, except that the amount of water removed out of the system by the second azeotropic distillation was changed to 100.9 g (277.5 g in total).

### <Example 3>

### [First-Stage Polymerization Step]

An 11 cm inner diameter, 2 L volume, cylindrical round-bottomed separable flask, which was equipped with a reflux condenser, a dropping funnel, a nitrogen gas inlet tube, and an agitator with stirring blades having two stages of four inclined paddle blades with a blade diameter of 5 cm, was prepared. As a hydrocarbon dispersion medium, 315 g of n-heptane was placed in this flask, and 0.782 g of a maleic anhydride-modified ethylene-propylene copolymer (Hi-Wax 1105A from Mitsui Chemicals, Inc.) was added as a polymeric dispersion agent. The mixture was heated with stirring to 80°C to dissolve the dispersion agent and then cooled to 50°C.

In a 300 mL inner volume beaker, 92.0 g (1.03 mol) of an 80.5% by mass aqueous solution of acrylic acid was placed as a water-soluble ethylenically unsaturated monomer, and 110.1 g of a 28.0% by mass aqueous solution of sodium hydroxide was added dropwise, with external cooling, to achieve 75 mol% neutralization. Then, 0.092 g (0.339 mmol) of 2,2'-azobis(2-amidinopropane) dihydrochloride and 0.028 g (0.102 mmol) of potassium persulfate as water-soluble radical polymerization initiators, 0.0101 g (0.058 mmol) of ethylene glycol diglycidyl ether as an internal-crosslinking agent, and 37.2 g of ion exchange water were added and dissolved to prepare a first-stage aqueous monomer solution.

Then, the aqueous solution prepared as above was added to the separable flask and stirred for 10 minutes. Then, a surfactant solution prepared by heating and dissolving 0.828 g of sucrose stearate having an HLB of 3 (Ryoto sugar ester S-370 from Mitsubishi-Kagaku Foods Corporation) as a surfactant in 7.45 g of n-heptane was further added. The system was sufficiently purged with nitrogen while stirring with the agitator at a rotation speed of 500 rpm. Then, the flask was immersed in a water bath at 70°C and heated, and polymerization was performed for 60 minutes to give a first-stage polymerization slurry containing primary particles.

### [Second-Stage Polymerization Step]

In a 500 mL inner volume beaker, 147.2 g (1.64 mol) of an 80.5% by mass aqueous solution of acrylic acid was placed as a water-soluble ethylenically unsaturated monomer, and 176.2 g of a 28.0% by mass aqueous solution of sodium hydroxide was added dropwise, with external cooling, to achieve 75 mol% neutralization. Then, 0.074 g (0.271 mmol) of 2,2'-azobis(2-amidinopropane) dihydrochloride and 0.044 g (0.163 mmol) of potassium persulfate as water-soluble radical polymerization initiators, and 7.41 g of ion exchange water were added and dissolved to prepare a second-stage aqueous monomer solution.

The separable flask system was cooled to 25°C while stirring with the agitator at a rotation speed of 1000 rpm. Then, the entire amount of the second-stage aqueous solution was added to the first-stage polymerization slurry, and the system was purged with nitrogen for 30 minutes. Then, the flask was again immersed in a water bath at 70°C and heated, and the polymerization reaction was performed for 60 minutes. A hydrous gel after the second-stage polymerization was obtained by the procedure described above.

### [Surface-Crosslinking]

The flask was then immersed in an oil bath set at 125°C to remove 191.4 of water out of the system by azeotropic distillation of the water and n-heptane, while refluxing the n-heptane. Then, 5.32 g of a 4.5% by mass aqueous solution of diethylenetriaminepentaacetic acid pentasodium salt and 2.79 g of a 3% by mass aqueous solution of sodium sulfite were added to the flask with stirring to give a second-stage polymerization slurry containing secondary particles.

The flask was then again immersed in an oil bath set at 125°C to further remove 84.6 g (276.0 g in total) of water out of the system by azeotropic distillation of the water and n-heptane, while refluxing the n-heptane. Then, 5.98 g (0.69 mmol) of a 2% by mass aqueous solution of ethylene glycol diglycidyl ether was added to the flask as a surface-crosslinking agent, and the mixture was maintained at 83°C for 2 hours.

Then, the mixture was dried by evaporating the n-heptane at 125°C to give a dried product. The dried product was passed through a sieve with a mesh size of 850 µm, and the resulting dried product was mixed with 0.2% by mass of amorphous silica (Tokusil NP-S from Oriental Silicas Corporation) to give 223.3 g of water-absorbent resin particles of Example 3.

### <Comparative Example 1>

### [First-Stage Polymerization Step]

An 11 cm inner diameter, 2 L volume, cylindrical round-bottomed separable flask, which was equipped with a reflux condenser, a dropping funnel, a nitrogen gas inlet tube, and an agitator with stirring blades having two stages of four inclined paddle blades with a blade diameter of 5 cm, was prepared. As a hydrocarbon dispersion medium, 292 g of n-heptane was placed in this flask, and 0.736 g of a maleic anhydride-modified ethylene-propylene copolymer (Hi-Wax 1105A from Mitsui Chemicals, Inc.) was added as a polymeric dispersion agent. The mixture was heated with stirring to 80°C to dissolve the dispersion agent and then cooled to 50°C.

In a 300 mL inner volume beaker, 92.0 g (1.03 mol) of an 80.5% by mass aqueous solution of acrylic acid was placed as a water-soluble ethylenically unsaturated monomer, and 110.1 g of a 28.0% by mass aqueous solution of sodium hydroxide was added dropwise, with external cooling, to achieve 75 mol% neutralization. Then, 0.074 g (0.272 mmol) of potassium persulfate as a water-soluble radical polymerization initiator, 0.0101 g (0.058 mmol) of ethylene glycol diglycidyl ether as an internal-crosslinking agent, and 37.3 g of ion exchange water were added and dissolved to prepare a first-stage aqueous monomer solution.

Then, the aqueous solution prepared as above was added to the separable flask and stirred for 10 minutes. Then, a surfactant solution prepared by heating and dissolving 0.736 g of sucrose stearate having an HLB of 3 (Ryoto sugar ester S-370 from Mitsubishi-Kagaku Foods Corporation) as a surfactant in 7.45 g of n-heptane was further added. The system was sufficiently purged with nitrogen while stirring with the agitator at a rotation speed of 500 rpm. Then, the flask was immersed in a water bath at 70°C and heated, and polymerization was performed for 60 minutes to give a first-stage polymerization slurry containing primary particles.

### [Second-Stage Polymerization Step]

In a 500 mL inner volume beaker, 128.8 g (1.44 mol) of an 80.5% by mass aqueous solution of acrylic acid was placed as a water-soluble ethylenically unsaturated monomer, and 154.2 g of a 28.0% by mass aqueous solution of sodium hydroxide was added dropwise, with external cooling, to achieve 75 mol% neutralization. Then, 0.103 g (0.381 mmol) of potassium persulfate as a water-soluble radical polymerization initiator, 0.012 g (0.067 mmol) of ethylene glycol diglycidyl ether as an internal-crosslinking agent, and 6.48 g of ion exchange water were added and dissolved to prepare a second-stage aqueous monomer solution.

The separable flask system was cooled to 25°C while stirring with the agitator at a rotation speed of 1000 rpm. Then, the entire amount of the second-stage aqueous solution was added to the first-stage polymerization slurry, and the system was purged with nitrogen for 30 minutes. Then, the flask was again immersed in a water bath at 70°C and heated, and the polymerization reaction was performed for 60 minutes. A hydrous gel after the second-stage polymerization was obtained by the procedure described above.

### [Surface-Crosslinking]

The flask was then immersed in an oil bath set at 125°C to remove 176.6 g of water out of the system by azeotropic distillation of the water and n-heptane, while refluxing the n-heptane. Then, 4.91 g of a 4.5% by mass aqueous solution of diethylenetriaminepentaacetic acid pentasodium salt and 2.58 g of a 3% by mass aqueous solution of sodium sulfite were added to the flask with stirring to give a second-stage polymerization slurry containing secondary particles.

The flask was then again immersed in an oil bath set at 125°C to further remove 89.6 g (266.3 g in total) of water out of the system by azeotropic distillation of the water and n-heptane, while refluxing the n-heptane. Then, 4.42 g (0.51 mmol) of a 2% by mass aqueous solution of ethylene glycol diglycidyl ether was added to the flask as a surface-crosslinking agent, and the mixture was maintained at 83°C for 2 hours.

Then, the mixture was dried by evaporating the n-heptane at 125°C to give a dried product. The dried product was passed through a sieve with a mesh size of 850 µm, and the resulting dried product was mixed with 0.5% by mass of amorphous silica (Tokusil NP-S from Oriental Silicas Corporation) to give 227.3 g of water-absorbent resin particles of Comparative Example 1.

### <Comparative Example 2>

225.8 g of water-absorbent resin particles of Comparative Example 2 were obtained as in Example 1, except that in the second-stage polymerization step, the amount of ethylene glycol diglycidyl ether added as an internal-crosslinking agent was changed to 0.026 g (0.148 mmol), and the amount of ion exchange water added was changed to 6.46 g; the amount of water removed out of the system by the second azeotropic distillation was changed to 97.4 g (274.1 g in total), and the amount of the 2% by mass aqueous solution of ethylene glycol diglycidyl ether added as a surface-crosslinking agent was changed to 5.52 g (0.63 mmol).

For the water-absorbent resin particles obtained in each of the examples and comparative examples, the physiological saline absorption capacity under a load of 0.69 kPa, the physiological saline retention capacity, the physiological saline absorption capacity, the values of non-pressurization DW after 3 and 10 minutes, the water absorption rate (Vortex method), the median particle size, and the absorption time and re-wet amount of the absorbent article were measured by the following methods. The measurement was performed in an environment of 25°C ± 2°C and a humidity of 50 ± 10%, unless otherwise stated. The results are shown in Table 1.

### [Physiological Saline Absorption Capacity Under a Load of 0.69 kPa]

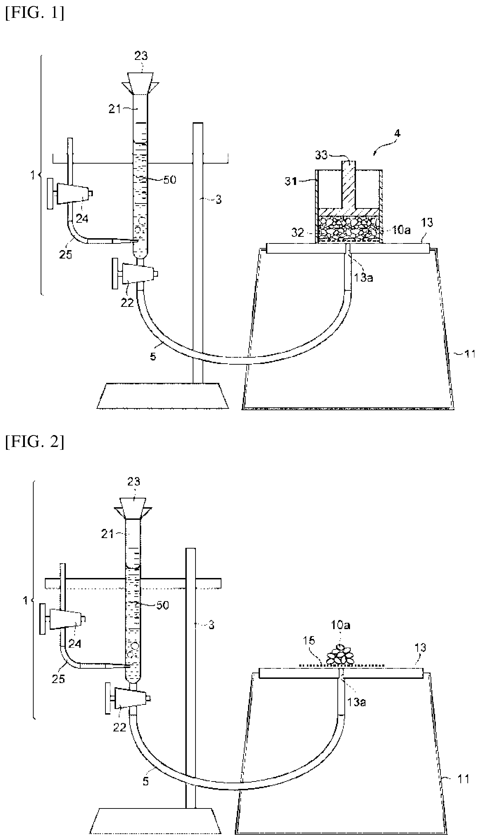

The water absorption capacity with respect to physiological saline under a load of 0.69 kPa (water absorption capacity under a load of 0.69 kPa) of the water-absorbent resin particles was measured using the apparatus schematically shown in Fig. 1. The water absorption capacity under the load was measured twice for one type of water-absorbent resin particles, and the average value of the measured values was obtained. The apparatus shown in Fig. 1 includes a burette unit 1, a clamp 3, a conduit 5, a stand 11, a measurement table 13, and a measurement unit 4 placed on the measurement table 13. The burette unit 1 has a graduated burette tube 21, a rubber stopper 23 for sealing the upper opening of the burette tube 21, a cock 22 connected to the lower end of the burette tube 21, an air inlet tube 25 connected to a lower portion of the burette tube 21, and a cock 24. The burette unit 1 is fixed by the clamp 3. The flat measurement table 13 has a through-hole 13a with a diameter of 2 mm formed in the center portion thereof, and is supported by the variable-height stand 11. The through-hole 13a of the measurement table 13 and the cock 22 of the burette unit 1 are connected through the conduit 5. The conduit 5 has an inner diameter of 6 mm.

The measurement unit 4 includes a cylinder 31 made of acrylic resin, a polyamide mesh 32 bonded to one opening of the cylinder 31, and a weight 33 vertically movable within the cylinder 31. The cylinder 31 is placed on the measurement table 13 with the polyamide mesh 32 therebetween. The cylinder 31 has an inner diameter of 20 mm. The polyamide mesh 32 has a mesh size of 75 µm (200 mesh). The weight 33 has a diameter of 19 mm and a mass of 20.5 g and, as described later, can apply a load of 0.69 kPa to water-absorbent resin particles 10a uniformly disposed on the polyamide mesh 32.

First, the cock 22 and the cock 24 of the burette unit 1 were closed, and 0.9% by mass physiological saline adjusted to 25°C was placed in the burette tube 21 through the upper opening of the burette tube 21. Next, the upper opening of the burette tube 21 was sealed with the rubber stopper 23, and then the cock 22 and the cock 24 were opened. The conduit 5 was filled with the 0.9% by mass saline 50 so as to prevent air bubbles from entering. The height of the measurement table 13 was adjusted so that the height of the surface of the 0.9% by mass saline reached into the through-hole 13a was equal to the height of the upper surface of the measurement table 13. After the adjustment, the height of the surface of the 0.9% by mass saline 50 in the burette tube 21 was read on the graduations of the burette tube 21, and this position was determined as a zero point (reading at 0 seconds).

In the measurement unit 4, 0.10 g of the water-absorbent resin particles 10a were uniformly disposed on the polyamide mesh 32 in the cylinder 31, the weight 33 was disposed on the water-absorbent resin particles 10a, and the cylinder 31 was placed such that the center portion thereof aligned with the conduit opening at the center portion of the measurement table 13. An amount of reduction Wa (mL) in the physiological saline in the burette tube 21 at 15 minutes after the beginning of absorption of the physiological saline by the water-absorbent resin particles 10a through the conduit 5 (that is, the amount of the physiological saline absorbed by the water-absorbent resin particles 10a) was read, and the physiological saline absorption capacity under a load of 0.69 kPa of the water-absorbent resin particles 10a was calculated using the following equation. The results are shown in Table 1. Physiological saline absorption capacity (mL/g) under a load of 0.69 kPa = Wa (mL)/mass (g) of the water-absorbent resin particles

### [Physiological Saline Retention Capacity]

2.0 g of the water-absorbent resin particles were weighed into a cotton bag (Cottonbroad No. 60, 100 mm in width × 200 mm in length), and the cotton bag was placed in a 500 mL beaker. 500 g of a 0.9% by mass aqueous solution of sodium chloride (physiological saline) was poured at once into the cotton bag containing the water-absorbent resin particles so as to prevent formation of unswollen lumps, then the top of the cotton bag was closed with a rubber band, and the cotton bag was allowed to stand for 30 minutes to cause the water-absorbent resin particles to swell. After 30 minutes, the cotton bag was dehydrated for 1 minute using a dehydrator (product number: H-122, manufactured by Kokusan Co., Ltd.) set at a centrifugal force of 167 G, and a mass Wb (g) of the cotton bag containing the swollen gel after dehydration was measured. The same procedure was performed without adding the water-absorbent resin particles, and an empty mass Wc (g) of the cotton bag upon wetting was measured. The physiological saline retention capacity was calculated using the following equation. The results are shown in Table 1. $Physiological saline retention capacity \left(g/g\right) = \left[Wb-Wc\right] / 2.0$

### [Physiological Saline Absorption Capacity]

500 g of 0.9% physiological saline was weighed into a 500 mL beaker. Next, 2.0 g of the water-absorbent resin particles were dispersed in the physiological saline while stirring at 600 rpm using a magnetic stirrer bar (8 mm ϕ × 30 mm in length, no ring) so as to prevent formation of unswollen lumps. The particles were left under stirring for 60 minutes and sufficiently swollen to give a dispersion containing a swollen gel. Subsequently, the mass Wd [g] of a standard sieve with a mesh size of 75 µm was measured, and then the dispersion described above was passed through this standard sieve. Then, excess water was removed by inclining the sieve at an inclination angle of about 30 degrees with respect to the horizontal direction, and leaving the sieve in this state for 30 minutes. The mass We [g] of the sieve on which the swollen gel remained was measured, and the physiological saline absorption capacity [g/g] was determined using the following equation: $Physiological saline absorption capacity \left(g/g\right) = \left(We-Wd\right) / 2.0$

### [Measurement of Values of Non-Pressurization DW (Demand Wettability) after 3 and 10 Minutes]

Each of the values of non-pressurization DW after 3 and10 minutes of the water-absorbent resin particles was measured using the measurement apparatus shown in Fig. 2. Each of these values was measured five times for one type of water-absorbent resin particles, and the average value of three measured values excluding the minimum and maximum values was obtained.

The measurement apparatus includes a burette unit 1, a conduit 5, a measurement table 13, a nylon mesh sheet 15, a stand 11, and a clamp 3. The burette unit 1 has a graduated burette tube 21, a rubber stopper 23 for sealing the upper opening of the burette tube 21, a cock 22 connected to the lower end of the burette tube 21, an air inlet tube 25 connected to a lower portion of the burette tube 21, and a cock 24. The burette unit 1 is fixed by the clamp 3. The flat measurement table 13 has a through-hole 13a with a diameter of 2 mm formed in the center portion thereof, and is supported by the variable-height stand 11. The through-hole 13a of the measurement table 13 and the cock 22 of the burette unit 1 are connected through the conduit 5. The conduit 5 has an inner diameter of 6 mm.

First, the cock 22 and the cock 24 of the burette unit 1 were closed, and 0.9% by mass saline 50 adjusted to 25°C was placed in the burette tube 21 through the upper opening of the burette tube 21. The saline concentration of 0.9% by mass is the concentration based on the mass of the saline. The opening of the burette tube 21 was sealed with the rubber stopper 23, and then the cock 22 and the cock 24 were opened. The conduit 5 was filled with the 0.9% by mass saline 50 to prevent air bubbles from entering. The height of the measurement table 13 was adjusted so that the height of the surface of the 0.9% by mass saline reached into the through-hole 13a was equal to the height of the upper surface of the measurement table 13. After the adjustment, the height of the surface of the 0.9% by mass saline 50 in the burette tube 21 was read on the graduations of the burette tube 21, and this position was determined as a zero point (reading at 0 seconds).

The nylon mesh sheet 15 (100 mm × 100 mm, 250 mesh, thickness about 50 µm) was laid in the vicinity of the through-hole 13a on the measurement table 13, and a cylinder having an inner diameter of 30 mm and a height of 20 mm was placed on the center portion of the nylon mesh sheet. 1.00 g of water-absorbent resin particles 10a were uniformly distributed in this cylinder. Then, the cylinder was carefully removed to obtain a sample in which the water-absorbent resin particles 10a were dispersed in a circle shape in the center portion of the nylon mesh sheet 15. Subsequently, the nylon mesh sheet 15 on which the water-absorbent resin particles 10a were placed was moved so rapidly that the water-absorbent resin particles 10a did not dissipate, so that the center of the nylon mesh sheet 15 was at the position of the through-hole 13a, and then the measurement was started. The time when air bubbles were first introduced through the air inlet tube 25 into the burette tube 21 was defined as the start of absorption (0 seconds).

An amount of reduction in the 0.9% by mass saline 50 in the burette tube 21 (that is, the amount of the 0.9% by mass saline absorbed by the water-absorbent resin particles 10a) was sequentially read by units of 0.1 mL, and an amount of reduction Wf (mL) of the 0.9% by mass saline 50 was read 3 minutes after the start of absorption by the water-absorbent resin particles 10a and an amount of reduction Wg (mL) of the 0.9% by mass saline 50 was read 10 minutes after the start of absorption by the water-absorbent resin particles 10a. The value of non-pressurization DW after 3 minutes was determined from Wf using the following equation. The value of non-pressurization DW after 10 minutes was also determined from Wg using the following equation. The non-pressurization DW is the water absorption capacity per 1.00 g of the water-absorbent resin particles 10a. $Value of non - pressurization DW after 3 minutes \left(mL/g\right) = Wf / 1.00$ $Value of non - pressurization DW after 10 minutes \left(mL/g\right) = Wg / 1.00$

### [Water Absorption Rate (Vortex Method)]

The water absorption rate with respect to physiological saline of the water-absorbent resin particles was measured by the following procedure based on the Vortex method. First, 50 ± 0.1 g of physiological saline adjusted to a temperature of 25 ± 0.2°C in a thermostatic water bath was weighed into a 100 mL inner volume beaker. Next, the physiological saline was stirred with a magnetic stirrer bar (8 mm ϕ × 30 mm, no ring) at a rotation speed of 600 rpm to form a vortex. 2.0 ± 0.002 g of the water-absorbent resin particles were added at once into the physiological saline. The time (seconds) from the addition of the water-absorbent resin particles to the point in time at which the vortex on the liquid surface disappeared was measured. The measured time was determined as the water absorption rate of the water-absorbent resin particles.

### [Method of Measuring Median Particle Size]

10 g of the water-absorbent resin particles were screened using a continuous fully automated sonic vibration type screening measuring device (Robot Sifter RPS-205, manufactured by Seishin Enterprise Co., Ltd), JIS standard sieves with mesh sizes of 850 µm, 710 µm, 600 µm, 500 µm, 425 µm, 300 µm, 250 µm, and 150 µm, and a receiving tray. The mass of the particles remaining on each sieve was calculated as the mass percentage relative to the total mass. The mass percentage of the particles remaining on each sieve was integrated in descending order of particle size. In this manner, the relationship between the sieve mesh size and the cumulative value of the mass percentage of the particles remaining on each sieve was plotted on logarithmic probability paper. The plots on the probability paper were connected with straight lines, and a particle size corresponding to a 50% by mass cumulative mass percentage was determined as the median particle size (µm).

### [Production of Absorbent Material and Absorbent Article]

### (Absorbent Material of Mixed Dispersion, and Absorbent Article)

An airflow-type mixing apparatus (Pad Former, manufactured by O-tec Co., Ltd.) was used to uniformly mix 8.11 g of the water-absorbent resin particles and 12.38 g of ground pulp by air-laid papermaking to produce an absorbent core with a size of 12 cm × 40 cm. Next, upper and lower portions of the absorbent core were sandwiched between two sheets of tissue paper (basis weight: 16 g/m²) of the same size as the absorbent core, and the whole material was pressed by applying a load of 141 kPa for 30 seconds to produce an absorbent material. In addition, a hydrophilic air-through nonwoven fabric (Rengo Nonwoven Products) (basis weight: 21 g/m²) of the same size as the absorbent material was disposed on the upper surface of the absorbent material. From a diaper manufactured by Daio Paper Corporation (trade name: GOON Plus designed for sensitive skin, tape type, size L, purchased in 2022), the original top sheet and absorbent material were removed to obtain a back sheet having a pair of side gathers bonded to both ends in the width direction. The hydrophilic air-through nonwoven fabric and the absorbent material were placed on the back sheet with their center portions superposed on each other to obtain an absorbent article for evaluation. In the absorbent article, the water-absorbent resin particles had a basis weight of 169 g/m², and the ground pulp (hydrophilic fibers) had a basis weight of 258 g/m².

### (Absorbent Material of Sheet-Like Structure, and Absorbent Article)

Two sheets of a spunlace nonwoven fabric (Zhejiang Wangjin Nonwovens Co., Ltd., basis weight: 35 g/m²) were cut to a size of 14 cm × 42 cm and used as spunlace nonwoven fabrics 1 and 2.

A hot-melt coater (HALLYS Corporation, pump: Marshal 150, table: XA-DT, tank set temperature: 150°C, hose internal set temperature: 165°C, gun head set temperature: 170°C) was used to apply a total of 0.2 g of a hot-melt adhesive (Henkel Japan Ltd., ME-765E) to the spunlace nonwoven fabric 1 in the form of 14 straight lines at 10 mm intervals along the longitudinal direction of the spunlace nonwoven fabric. The applied pattern of the adhesive was a spiral stripe. During the application, the four outer peripheral sides (width: 1 cm) of the spunlace nonwoven fabric 1 were masked to avoid the application of the adhesive.

6.5 g of the water-absorbent resin particles were uniformly distributed over a region of 12 cm × 40 cm in the center portion of the surface of the spunlace nonwoven fabric 1 to which the hot-melt adhesive was adhered, thus obtaining a water-absorbent layer A.

A total of 0.2 g of the hot-melt adhesive was applied to the spunlace nonwoven fabric 2 in the form of 14 straight lines at 10 mm intervals. The applied pattern of the adhesive was a spiral stripe. 13.5 g of the water-absorbent resin particles were uniformly distributed over the center portion (a region of 12 cm × 40 cm) of the surface of the spunlace nonwoven fabric 2 to which the adhesive was adhered, thus obtaining a water-absorbent layer B.

An air-through nonwoven fabric with a size of 12 cm × 40 cm (Jinhan Non-Woven Fabric Co., Ltd., Guangzhou City; basis weight: 45 g/m²) was placed on the surface of the spunlace nonwoven fabric 1 over which the water-absorbent resin particles had been distributed. Then, both ends of the spunlace nonwoven fabrics 1 and 2 were aligned such that their adhesive-applied surfaces were on the inner side, these fabrics were sandwiched between release papers, and pressed using a laminating machine (HASHIMA CO., LTD., Straight Linear Fussing Press, model HP-600LFS) at 110°C and 0.1 MPa to laminate them to each other. The release papers were removed from the resulting material to obtain an absorbent material. The region where the water-absorbent resin particles were distributed in the absorbent material was 12 cm × 40 cm.

In addition, a hydrophilic air-through nonwoven fabric (Rengo Nonwoven Products) (basis weight: 21 g/m²) with a size of 12 cm × 40 cm was disposed on the upper surface of the absorbent material with their center portions superposed on each other. From a diaper manufactured by The P&G Japan Limited (trade name: Pampers Sarasara Care, tape type, size L, purchased in 2022), the original top sheet and absorbent material were removed to obtain a back sheet having a pair of side gathers bonded to both ends in the width direction. The hydrophilic air-through nonwoven fabric and the absorbent material were placed on the back sheet with their center portions superposed on each other to obtain an absorbent article for evaluation. In the absorbent article, the water-absorbent resin particles had a basis weight of 340 g/m². The absorbent article included the hydrophilic air-through nonwoven fabric, the water-absorbent layer A, the air-through nonwoven fabric, and the water-absorbent layer B disposed in this order.

### [Measurement of Absorption Time and Re-Wet Amount]

The absorption time and re-wet of a liquid of the absorbent article were measured in accordance with the Chinese GB standard "GB/T 28004.1-2021 NATIONAL STANDARD OF THE PEOPLE'S REPUBLIC OF CHINA, Disposable diapers- Part 1: Disposable diapers for baby". The specific method is described below.

A U-shaped sample base (Fig. A.1 of the GB standard), a sample holder (Fig. A.2 of the GB standard), a test liquid dosing module (Fig. A.3 of the GB standard), and a pressurization module (Fig. A.4 of the GB standard) were prepared. A test liquid was obtained by mixing 90.0 g of sodium chloride, 9910 g of deionized water, and Food Blue No. 1 (for coloring), and the test liquid was adjusted to a temperature of 36 ± 1 degrees in a thermostatic bath. The absorbent article described above was placed on the U-shaped sample base and the sample holder put on a horizontal table. The size of each jig was as follows.

U-shaped sample base: B2 L: 136 mm, W: 135 mm
Sample holder: T2
Test liquid dosing module: M2 W: 85 mm, L: 108 mm
Pressurization module: Y2 W: 85 mm, L: 108 mm

80 mL of the test liquid was added at a speed of 480 mL/min, using a liquid feed pump and the test liquid dosing module of Fig. A.3 of the GB standard. The time from the addition of the test liquid to the disappearance of the liquid surface at the lowest point of the absorption area was measured as a first absorption time (seconds). Here, the test liquid dosing module was adjusted to apply a pressure of 2.00 kPa to the sample surface, and the pressurization was continued for 1 minute from the liquid addition. After 1 minute, the test liquid dosing module was removed. When the absorption time was longer than 1 minute, the test liquid dosing module was removed after waiting until the completion of absorption. The first absorption test was thus completed.

8 minutes after the first addition of the test liquid, a second absorption time (seconds) was measured by the same procedure as in the measurement of the first absorption time. A sum of the first absorption time and the second absorption time was determined as the total absorption time (seconds). 15 minutes after the first addition of the test liquid, a stack of 10 sheets of 11.0 cm × 7.0 cm filter paper was placed on the center portion of the sample surface, with the center portion of the filter paper superposed on the center portion of the sample surface. 16 minutes after the first addition of the test liquid, the pressurization module was pressed against the filter paper to apply a pressure of 4.00 kPa. After 1 minute of pressurization, the pressurization module and the filter paper were removed, and the weight of the filter paper was measured with a balance. A difference in weight of the filter paper between before and after the test was calculated as the mass of the test liquid absorbed by the filter paper, which was determined as the re-wet amount (g).

**[Table 1]**

| | Water-absorbent resin particles | | | | | | | Absorbent article | | | |
|---|---|---|---|---|---|---|---|---|---|---|---|
| | | | | | | | | Mixed dispersion | | Sheet-like structure | |
| | Physiological saline absorption capacity under a load of 0.69 kPa | Physiological saline retention capacity | Physiological saline absorption capacity | Value of non-pressurization DW after 3 minutes | Value of non-pressurization DW after 10 minutes | Water absorption rate | Median particle size | Re-wet amount in a curved state and under pressure | Total absorption time | Re-wet amount in a curved state and under pressure | Total absorption time |
| | mL/g | g/g | g/g | mL/g | mL/g | sec | µm | g | sec | g | sec |
| Ex. 1 | 53 | 50 | 65 | 30 | 54 | 47 | 410 | 4 | 70 | 0 | 78 |
| Ex. 2 | 40 | 42 | 60 | 43 | 57 | 33 | 365 | 6 | 58 | - | - |
| Ex. 3 | 50 | 55 | 67 | 41 | 71 | 36 | 370 | 2 | 60 | - | - |
| Comp. Ex. 1 | 18 | 50 | 67 | 4 | 46 | 43 | 301 | 16 | 105 | - | - |
| Comp. Ex. 2 | 22 | 57 | 67 | 13 | 45 | 40 | 351 | 13 | 91 | - | - |

### Reference Signs List

1: burette unit
3: clamp
4: measurement unit
5: conduit
10a: water-absorbent resin particles
11: stand
13: measurement table
13a: through-hole
15: nylon mesh sheet
21: burette tube
22: cock
23: rubber stopper
24: cock
25: air inlet tube
31: cylinder
32: polyamide mesh
33: weight
50: saline

## Claims

1. Water-absorbent resin particles having a value of physiological saline absorption capacity under a load of 0.69 kPa after 15 minutes of 30 mL/g or more and 60 mL/g or less.

2. The water-absorbent resin particles according to claim 1, wherein the water-absorbent resin particles have a value of non-pressurization DW after 3 minutes of 20 mL/g or more and 50 mL/g or less.

3. The water-absorbent resin particles according to claim 2, wherein the water-absorbent resin particles have a water absorption rate with respect to physiological saline of 60 seconds or less.

4. The water-absorbent resin particles according to claim 2, wherein the water-absorbent resin particles have a physiological saline retention capacity of 30 g/g or more.

5. An absorbent material comprising the water-absorbent resin particles according to any one of claims 1 to 4.

6. An absorbent article comprising the absorbent material according to claim 5.
